(19) Europäisches Patentamt
European Patent Office
Office européen des brevets

(11) Numéro de publication: **0 087 359**
**B1**

(12) **FASCICULE DE BREVET EUROPÉEN**

(45) Date de publication du fascicule du brevet: **31.05.89**

(21) Numéro de dépôt: **83400315.4**

(22) Date de dépôt: **15.02.83**

(51) Int. Cl.⁴: **C 07 B 43/02,** C 07 C 76/02, C 07 C 79/02, C 07 J 41/00, C 07 J 5/00

(54) **Nouveau réactif de nitrométhylation, son application à la préparation de composés nitrométhylénés et de certains de leurs dérivés et les composés nouveaux obtenus.**

(30) Priorité: **18.02.82 FR 8202681**

(43) Date de publication de la demande: **31.08.83 Bulletin 83/35**

(45) Mention de la délivrance du brevet: **31.05.89 Bulletin 89/22**

(84) Etats contractants désignés: **AT CH DE FR GB IT LI NL SE**

(56) Documents cité:
**EP-A-0 023 856**
**GB-A-2 079 756**
**US-A-2 383 603**

**CHEMICAL ABSTRACTS, vol. 28, no. 15, 10 août 1934, colonnes 4700/3-4701/4, Columbus, Ohio, US H.B. FRASER et al.: "Effect of the nitro group in three-carbon tautomerism"**
**JOURNAL OF THE CHEMICAL SOCIETY, CHEMICAL COMMUNICATIONS, no. 15, 1er août 1981, pages 774-775, Londres, GB D.H.R BARTON et al.: "Efficient synthesis of the corticosteroid side-chain from 17-ketones"**
**JOURNAL OF THE CHEMICAL SOCIETY, CHEMICAL COMMUNICATIONS, vol. 10, mai 1982, pages 551-552, Londres, GB D.H.R. BARTON et al.: "A simple construction of the hydroxyketone side chain of corticosteroids from 17-oxo-steroids via**

(73) Titulaire: **ROUSSEL- UCLAF, 35, boulevard des Invalides, F-75007 Paris (FR)**

(72) Inventeur: **Barton, Derek Harold Richard, Bât. A/B/C/, Route du Château Fort, F-91190 Gif sur Yvette (FR)**
Inventeur: **Motherwell, William Branks Bât. 3, Appt. 41, Résidence des Quinconces Route du Château Fort, F-91190 Gif sur Yvette (FR)**
Inventeur: **Zard Zard, Samir, Appt. 96, 4, Résidence des Fonds Fanettes, F-91190 Gif sur Yvette (FR)**

(74) Mandataire: **Tonnellier, Marie- José, Département des Brevets ROUSSEL UCLAF B.P. no 9, F-93230 Romainville (FR)**

(56) Documents cité: (suite)
**nitro-olefins"**

Il est rappelé que: Dans un délai de neuf mois à compter de la date de publication de la mention de la délivrance du brevet europeen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



LIBER, STOCKHOLM 1989

## Description

La présente invention concerne un réactif de nitrométhylation, son application à la préparation de composés nitrométhylénés et de certains de leurs dérivés et les composés obtenus.

L'invention a ainsi pour objet un réactif de nitrométhylation de composés cétoniques caractérisé en ce qu'il comprend le nitrométhane et un catalyseur basique bifonctionnel choisi parmi l'éthylène diamine, la triméthylène diamine et la tétraméthylène diamine.

Le nitrométhane est un produit chimique connu; on vient de découvrir que le nitrométhane utilisé en présence d'un catalyseur basique bifonctionnel était un réactif de nitrométhylation, pouvant être utilisé notamment dans la synthèse de composés cortisoniques préparés à partir de 17 céto stéroïdes.

On connaissait des voies d'accés à ces composés cortisoniques à partir de composés 17 céto stéroïdes, voir notamment EP-A-0 023 856, GB-A-2 079 756 et Journal of the Chemical Society chem. Communication n° 15, 1er août 1981, pages 774 - 775. On vient de découvrir une nouvelle voie d'accès mettant en oeuvre le réactif de l'invention.

Par réaction de nitrométhylation des composés cétoniques on entend les réactions permettant de transformer un groupe cétonique porté par un composé organique cyclique ou acyclique en un groupe nitrométhylène. On peut citer à titre d'illustration, une réaction du type:

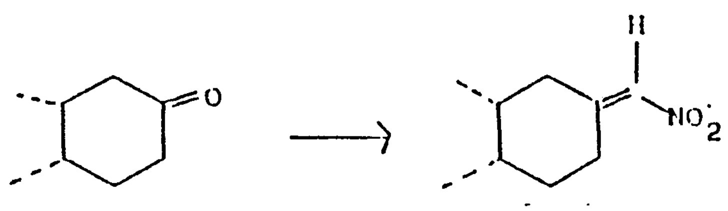

L'invention a également pour objet l'application du réactif tel que défini précédemment, caractérisée en ce que l'on soumet un composé organique cyclique ou acyclique, comportant au moins un groupe cétonique, à l'action dudit réactif et obtient ainsi un composé nitrométhyléné.

Dans des conditions préférentielles d'exécution de l'application ci-dessus, la réaction est effectuée en l'absence de solvant.

L'invention a notamment pour objet une application telle que définie ci-dessus, caractérisée en ce que le composé cétonique répond à la formule (II):

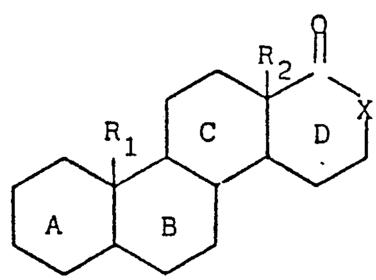

(II)

dans laquelle $R_1$ représente un atome d'hydrogène, un radical alkyle renfermant de 1 à 4 atomes de carbone éventuellement substitué par une fonction oxygénée ou azotée ou par un atome d'halogène ou $R_1$ représente un radical alkenyle ou alkynyle renfermant de 2 à 4 atomes de carbone, $R_2$ représente un radical alkyle renfermant de 1 à 4 atomes de carbone, X représente un atome de carbone ou une liaison carbone-carbone, les noyaux A, B, C et D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle et alkynyle renfermant de 2 à 4 atomes de carbone, et que l'on obtjent le composé de formule (I) correspondant:

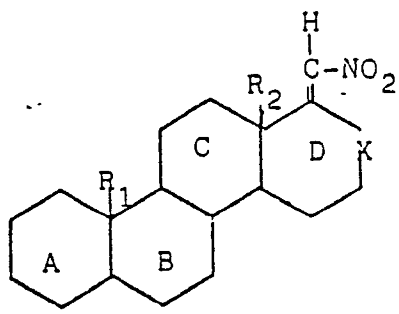

(I)

dans laquelle $R_1$, $R_2$, X, A, B, C et D sont définis comme précédemment.

Dans les composés de formules (II) et (I), $R_1$, $R_2$, A, B, C et D ont de préférence les significations suivantes:

Lorsque $R_1$ représente un radical alkyle, il s'agit de préférence du radical méthyle ou éthyle.

Lorsque $R_1$ représente un radical alkyle substitué par une fonction oxygénée, il s'agit de préférence du radical hydroxy méthyle ou hydroxy éthyle, du radical formyle ou du radical acétyle.

Lorsque $R_1$ représente un radical alkyle substitué par une fonction azotée, il s'agit de préférence du radical cyano ou du radical amino méthyle ou amino éthyle.

Lorsque $R_1$ représente un radical alkyle substitué par un halogène, il s'agit de préférence d'un radical $-CH_2Hal$, dans lequel Hal représente un atome d'halogène, comme, par exemple, un atome de chlore, de fluor ou de brome.

Lorsque $R_1$ représente un radical alkényle, il s'agit de préférence du radical vinyle ou allyle.

Lorsque $R_1$ représente un radical alkynyle, il s'agit de préférence du radical éthynyle.

$R_2$ représente de préférence un radical méthyle ou éthyle.

Lorsque les noyaux A, B, C et D portent une ou plusieurs doubles liaisons, il s'agit de préférence de doubles liaisons en 1(2), 3(4), 4(5) ou 9(11) ou d'un système de doubles liaisons conjuguées en 3(4) et 5(6) ou en 4(5) et 6(7) ou en 1(2) et 4(5) ou d'un système aromatique de trois doubles liaisons 1, 3, 5 ou d'un système de trois doubles liaisons 1(2), 4(5), 6(7).

Lorsque les cycles A, B, C et D sont substitués par une ou plusieurs fonctions hydroxyles, il s'agit de préférence d'une fonction hydroxyle en 3 ou en 11.

Lorsque les cycles A, B, C et D sont substitués par une ou plusieurs fonctions cétones, il s'agit de préférence d'une fonction cétone en 3 ou en 11.

Lorsque les cycles A, B, C et D sont substitués par un ou plusieurs atomes d'halogènes, il s'agit de préférence d'une atome de fluor, de chlore ou de brome en position 6 ou 9 $\alpha$ par exemple.

Lorsque les cycles A, B, C et D sont substitués par un ou plusieurs radicaux alkyles, il s'agit de préférence du radical méthyle ou éthyle en 2, 6, 7 en 16 $\alpha$ ou en 16 $\beta$.

Lorsque les cycles A, B, C et D sont substitués par un ou plusieurs radicaux alkyloxy, il s'agit de préférence d'un radical méthoxy ou éthoxy en 3 ou 11 $\beta$.

Lorsque les cycles A, B, C et D sont substitués par un ou plusieurs radicaux alkényles, il s'agit de préférence du radical vinyle ou allyle en position 11 $\beta$ par exemple. Lorsque les cycles A, B, C et D sont substitués par un ou plusieurs radicaux alkynyles, il s'agit de préférence du radical éthynyle en position 11 $\beta$ par exemple.

L'invention a notamment pour objet une application telle que définie précédemment, caractérisée en ce que le composé cétonique répond à la formule ($II_A$):

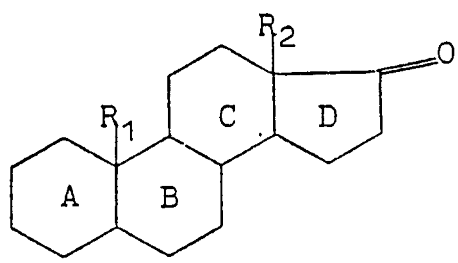

($II_A$)

dans laquelle $R_1$, $R_2$, A, B, C et D sont définis comme précédemment et que l'on obtient le composé de formule ($I_A$) correspondant:

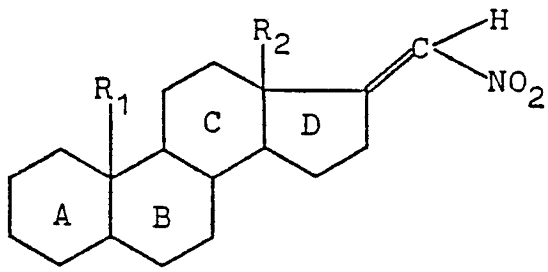

$(I_A)$

dans laquelle $R_1$, $R_2$, A, B, C et D sont définis comme précédemment.

L'invention a particulièrement pour objet une application telle que définie précédemment, caractérisée en ce que le composé cétonique répond à la formule $(II_A)$ dans laquelle $R_2$ représente un radical méthyle, ainsi qu'à la formule $(II_A)$ dans laquelle $R_1$ représente un atome d'hydrogène ou un radical méthyle et que l'on obtient les composés de formule $(I_A)$ correspondants.

L'invention a plus particulièrement pour objet une application telle que définie précédemment, caractérisée en ce que le composé cétonique répond à la formule (II'A):

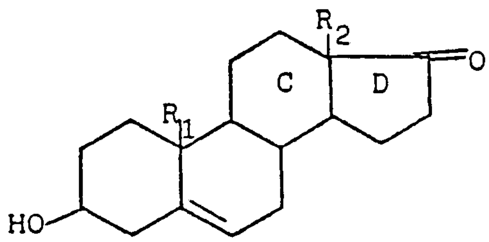

$(II'_A)$

dans laquelle $R_1$, $R_2$, C et D sont définis comme précédemment, et que l'on obtient le composé de formule $(I'_A)$ correspondant:

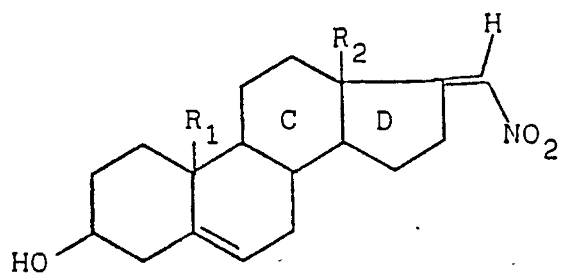

$(I'_A)$

dans laquelle $R_1$, $R_2$, C et D sont définis comme précédemment, ainsi qu'une application telle que définie précédemment, caractérisée en ce que le composé cetonique repond a la formule $(II''_A)$:

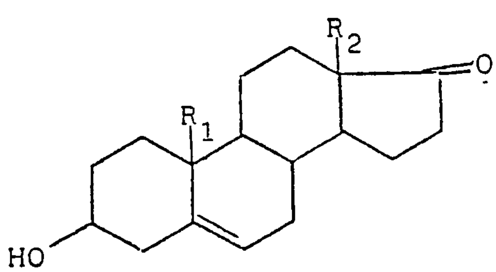

(II"A)

dans laquelle $R_1$ et $R_2$ sont définis comme précédemment, et que l'on obtient le composé de formule $(I''_A)$ correspondant:

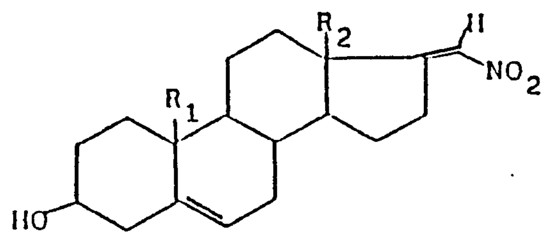

(I"$_A$)

dans laquelle $R_1$ et $R_2$ sont définis comme précédemment.

L'invention a tout particulièrement pour objet une application telle que définie précédemment, caractérisée en ce que le produit de formule II utilsé est la 3 β-hydroxy androst-5-em-1-one et le produit de formule I obtenu est le 3 β-hydroxy 17-(nitrométhylène)androst-5-ène.

Comme indiqué précédemment l'application selon l'invention concerne les composés organiques cycliques ou acycliques en général et n'est, bien entendu, pas limitée aux composés stéroïdes.

La partie expérimentale ci-après fournit un autre exemple d'application à la préparation d'un composé bicyclique nitrométhylé, au départ du composé cétonique correspondant.

Les composés de formule (I) obtenus par l'application selon l'invention sont nouveaux.

On peut citer notamment, parmi ceux-ci, les composes de formule $I_A$ et plus particulièrement ceux pour lesquels $R_2$ represente un radical méthyle et ceux pour lesquels $R_1$ représente un atome d'hydrogène ou un radical méthyle. On peut également citer comme composés tout spécialement visés, les composés répondant à la formule (I'$_A$):

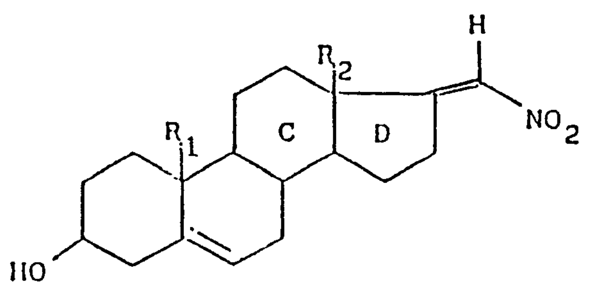

(I'$_A$)

dans laquelle $R_1$, $R_2$, C et D sont définis comme précédemment et plus particulièrement, parmi ceux-ci les composés répondant à la formule (I"$_A$):

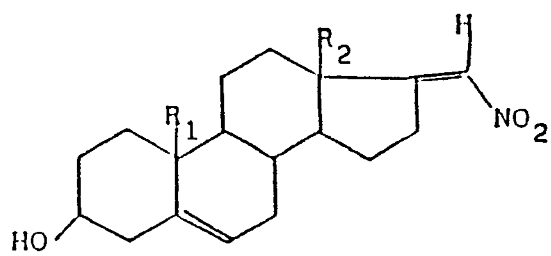

(I"$_A$)

dans laquelle $R_1$ et $R_2$ sont définis comme précédemment et tout particulièrement, le composé de formule I dont la préparation est donnée plus loin dans la partie expérimentale.

Les produits de formule I et notamment ceux de formule $I_A$ sont d'un très grand intérêt industriel. Ils sont en effet préparés directement avec d'excellents rendements à partir des composés 17-cétoniques correspondants par un procédé simple et économique; ils peuvent être facilement transformés en composés 21-acétoxy 20-oxo corticostéroïdes portant ou non une double liaison en 16(17), sans qu'il soit nécessaire d'isoler les produits intermédiaires obtenus.

Les composés de formulee $I_A$ peuvent servir notamment à préparer les produits décrits dans la demande de brevet allemand 2 521 231, dans les brevets français 1 058 850 et 1 021 728 dans le brevet américain 3 445 490.

Les produits de formule I et notamment ceux de formule $I_A$ peuvent donc être utilisés dans la préparation de produits représéntant un très grand intérêt comme médicaments, comme, par exemple, la triamcinolone et ses dérivés.

Les exemples suivants illustrent i'invention.

**Exemple 1**

3 β-hydroxy 17-(nitrométhylène)androst-5-ène

On chauffe au reflux sous atmosphère d'azote, une solution renfermant 8,7 g de 3β-hydroxy androst-5-èn. 17-one dans 150 $cm^3$ de nitrométhane, pour distiller 10 $cm^3$ de nitrométhane. On ajoute 0,1 $cm^3$ d'éthylènediamine et on chauffe le mélange au reflux pendant 50 - 60 heures. On chasse le solvant sous pression réduite, reprend le résidu dans le dichlorométhane et le filtre sur silice en éluant par le mélange éther-dichlorométhane (1/1). On chasse le solvant organique sous pression réduite. On obtient ainsi 9,9 g du produit recherché fondant à 118 - 122°C. Le produit cristallise avec une demie-mole de méthanol. F = 165 = 168°C.

$(\alpha)_D$ = - 88° (c = 0,76 % méthanol).

RMN $CCl_4$ ppm

6,65: 1H large 20 H

5,25: 1H large 6 H

1,05: 3H singulet 10 $CH_3$

0,95: 3H singulet 13 $CH_3$

**Exemple 2**

3,4-dihydro 2-nitrométhane naphtalène

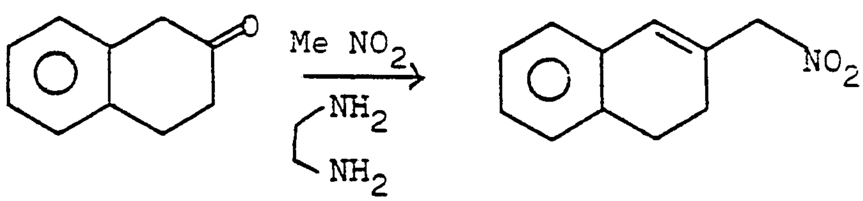


Une solution de 220 mg de α-tétralone dans 3 $cm^3$ de nitrométhane contenant environ 15 mg d'éthylène diamine, est chauffée à 75°C sous azote pendant 18 heures. L'excès de nitrométhane est évaporé sous pression reduite et le residu est chromatographié sur silice (benzène-pentane 2/3). On obtient 232 mg de produit brut que l'on recristallise dans le pentane, F = 29 - 31°C. Spectre IR : max 1550 $cm^{-1}$,

RMN $CDCl_3$ ppm

6,9: (4H, aromatiques)

6,4: (1H, singulet large, H éthylénique)

4,9: (2H, singulet $-CH_2-N_2$)

2,3 - 3,0: (4H, m, $-CH_2$)

Analyse

| | | | | | | |
|---|---|---|---|---|---|---|
| Trouvé : | C % | 69,96 | H % | 5,88 | N % | 7,49 |
| Calculé : | | 69,83 | | 5,86 | | 7,40 |

Application 1 : 3β,21-diacétoxy-prégna 5,16-dièn 20-one

Stade A: 3β,21-dihydroxy 20-nitro prégna 5,16-diène et diacétate correspondant

On maintient sous agitation à la température ambiante pendant 30 minutes, un mélange renfermant une suspension de 500 mg de 3β-hydroxy 17-(nitrométhylène) androst 5-ène dans 10 $cm^3$ d'isopropanol, 2 $cm^3$ d'une solution aqueuse de formaldéhyde et 1 $cm^3$ de triéthylamine. On verse le mélange réactionnel dans une solution aqueuse d'acide acétique. On agite pendant 30 minutes, filtre et sèche le produit obtenu. On obtient ainsi 511 mg de produit recherché sous forme de diacétate qui fond à 155 - 163°C.

$(\alpha)_D$ = -52° (c = 0,48 % $CHCl_3$)

RMN $CCl_4$ ppm

5,95 : 1H large 16H

5,30 : 1H large 6H

4,9 - 5,1 : 1H large 20H

4,1 - 4,6 : 3H large 3H et 21H

2,05 et 1,97 : 6H, singulet acétate

1,10 : 3H, singulet 10-$CH_3$

0,85 : 3H, singulet 13-$CH_3$

Stade B : 20-oxiome de 3β-21-diacétoxy prégna 5,16-dièn20-one.

On ajoute 6 $cm^3$ d'une solution d'acide chlorhydrique concentré et 1,8 g de poudre de zinc à une solution

renfermant 3 g de $CrCl_3$-$6H_2O$ dans 14 $cm^3$ d'eau. On filtre la solution obtenue et la verse dans une solution renfermant 1 g de 3β,21-diacétoxy 20-nitro prégna 5,16-diène dans 150 $cm^3$ d'acétone. Après 6 heures d'agitation, on verse la solution obtenue dans une solution aqueuse de chlorure de sodium et extrait à l'éther. On lave la phase éthérée à l'eau salée et la sèche. On filtre sur silice, evapore à sec et obtient 830 mg d'un solide blanc cristallisé fondant à 173 - 176°C.

$(\alpha)_D$ = -42° (c = 1,06 % $CHCl_3$)

<u>RMN</u> $CCl_4$ ppm

5,97: 1H large 16 H
5,27: 1H large 6 H
4,88: 2H, singulet 21-$CH_2$
4,3 - 4,7: 1H large 3H
2,04 et 2,00: 6H, singulet acétate
1,05: 3H, singulet 10-$CH_3$
0,94: 3H, singulet 13-$CH_3$

<u>Stade C</u> : 3β,21-diacétoxy prégna 5,16-dièn 20-one

On ajoute 6,5 $cm^3$ d'une solution aqueuse de trichlorure de titane dans une suspension renfermant 800 mg de 20-oxime de 3 β,21-diacétoxy prégna 5,16-dièn-20-one, 16 $cm^3$ d'acide acétique et 6 $cm^3$ d'acétone contenant 2,4 g d'acétate d'ammonium. On maintient le mélange réactionnel sous agitation pendant 6 heures, le verse dans l'eau, et extrait à l'éther. On lave la phase éthérée et la sèche. On filtre sur silice. On obtient après évaporation 766 mg d'un solide blanc fondant à 154 - 156°C.

$(\alpha)_D$ = -39° (c = 0,95 % $CHCl_3$).

<u>Aplication 2 : Préparation de la 3β,21-diacétoxy prégn-5-en 20-one</u>

1) <u>17β-nitrométhyl 3β-hydroxy androst-5-ène</u>

A une suspension de 0,5 g de 17-nitrométhylène 3β-hydroxy androst-5-ène dans 15 $cm^3$ d'isopropanol, on ajoute peu à peu en 10 minutes, environ 100 mg de borohydrure de sodium. On agite le mélange à température ambiante pendant 2 heures, verse dans 300 $cm^3$ d'une solution aqueuse d'acide acétique à 1 %. On filtre, sèche et obtient 0,5 g de produit attendu.

F = 178 - 181°C (méthanol).
$(\alpha)_D$ = -70° (c = 1 % $CHCl_3$)
<u>Spectre IR</u> : max 1540 $^{cm-1}$
<u>RMN</u> $CDCl_3$ ppm
5,25: (1H, m, 6-H)
4,25: (2H, m, 20-$CH_2$)
3,4: (1H, large, 3α-H)
1,05: (3H, singulet, 19-$CH_3$)
0,7: (3H, singulet, 18-$CH_3$)

2) <u>3β,21-diacétoxy 20-nitro-prégn-5-ène</u>

A une suspension de 1 g de 17β-nitrométhyl 3β-hydroxy androst-5-ène dans 20 $cm^3$ d'isopropanol, on ajoute 4 $cm^3$ de formol et 2 $cm^3$ de triéthylamine. On agite à température ambiante pendant 40 - 50 minutes puis verse dans 200 $cm^3$ d'une solution aqueuse d'acide acétique à 2,5 %. On filtre, sèche et obtient 1,07 g de produit attendu.

F = 187 - 195°C (méthanol).
$(\alpha)_D$ = -22° (c = 0,4 % $CHCl_3$).

3) <u>3β,21-diacétoxy-prégn-5-èn-20-one</u>

A une solution de 200 mg de 3β,21-diacétoxy 20-nitro-prégn-5-ène dans 35 $cm^3$ d'acétone on ajoute sous azote une solution filtrée de chlorure chromeux (préparée à partir de 0,6 g de $CrCl_3$-6 $H_2O$ et de 0,3 g de zinc en poudre dans une solution aqueuse d'acide chlorhydrique renfermant 1,5 $cm^3$ d'acide concentré et 2,5 $cm^3$ d'eau). Après 2 - 3 minutes, on verse le mélange, dans l'eau et extrait avec du dichlorométhane. La phase organique est séchée et évaporée et le résidu est dissous dans 4 $cm^3$ d'acide acétique. On ajoute goutte à goutte 2,5 $cm^3$ d'une solution aqueuse de nitrite de sodium, puis 4 $cm^3$ d'acide acétique. Le mélange est bouilli pendant 10 minutes, versé dans l'eau, neutralisé par le carbonate de sodium et extrait avec le dichlorométhane. La phase organique est séchée, filtrée sur silice et évaporée pour donner 149 mg de produit attendu.

F = 162 - 164° C
$(\alpha)_D$ = + 22° (c = 0,7 % $CHCl_3$)
(Lit. (1) F = 166 - 168°C; $(\alpha)_D$ = +27°)
(1) 1. Marquet et J. Jacques, Bull Soc. Chim. Fr. 1962, 90.

**Exemple 3**

3-méthoxy 17-(nitrométhylène)19-nor androsta 1,3,5-triène

On opère de manière analogue à celle décrite à l'exemple 1, en utilisant au départ 0,2 g d'éther méthylique de l'oestrone, 5 $cm^3$ de nitrométhane et environ 5 mg d'éthylène diamine. On obtient après chromatographie du produit brut obtenu, sur silice, en éluant au mélange hexane-dichlorométhane (2 - 3), 0,146 g de produit attendu.

F = 192 - 205°C.

$(\alpha)^{20}_D = +10°$ ( c = 0,78 - $CHCl_3$)

**Exemple 4**

3-nitrométhyl cholest-2-ène

On mélange 0,1 g de cholestanone, 3 $cm^3$ de nitrométhane et 2 mg d'éthylène diamine. On porte au reflux pendant une heure, élimine le solvant sous pression réduite à température ambiante et obtient 0,11 g de produit brut, que l'on recristallise dans le méthanol. On obtient le produit attendu.

F = 108 - 112°C.

$(\alpha)^{20}_D = +66°$ (c = 0,56 $CHCl_3$)

**Exemple 5**

2-nitrométhylène adamantane

On mélange 0,5 g de 2-adamantone, 15 $cm^3$ de nitrométhane et 20 mg d'éthylène diamine, porte au reflux pendant trois heures sous atmosphère d'azote, élimine le solvant sous pression réduite à température ambiante et dissout le résidu dans le chlorure de méthylène. On fait passer la solution sur silice et évapore le solvant. On obtient 0,63 g de produit attendu.

F = 78 - 81°C.

**Revendications** pour les Etats contractants: CH, DE, FR, GB, IT, LI, NL, SE

1. Réactif de nitrométhylation de composés cétoniques caractérisé en ce qu'il comprend le nitrométhane et un catalyseur basique bifonctionnel choisi parmi l'éthylène diamine, la triméthylène diamine et la tétraméthylène diamine.

2. Application du réactif tel que défini à la revendication 1 ou 2, caractérisée en ce que l'on soumet un composé organique cyclique ou acyclique, comportent au moins un groupe cétonique, à l'action dudit réactif, et obtient ainsi un composé nitrométhyléné.

3. Application selon la revendication 2, caractérisée en ce que le composé cétonique répond à la formule (II):

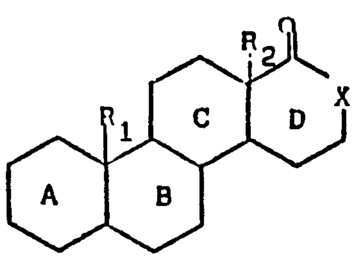

(II).

dans laquelle $R_1$ représente un atome d'hydrogène, un radical alkyle renfermant de 1 à 4 atomes de carbone éventuellement substitué par une fonction oxygénée ou azotée ou par un atome d'halogène, ou $R_1$ représente un radical alkényle ou alkynyle renfermant de 2 à 4 atomes de carbone, $R_2$ représente un radical alkyle renfermant de 1 à 4 atomes de carbone, X représente un atome de carbone ou une liaison carbone-carbone, les moyaux A, B, C et D portent éventuellement une ou plusieurs doubles liaisons et sont éventuellememt substitués par une ou plusieurs fonctions hydroxyle ou cétone, par un ou plusieurs atomes d'halogène, par un ou plusieurs radicaux alkyle ou alkyloxy renfermamt de 1 à 4 atomes de carbone ou par um ou plusieurs radicaux alkényle et alkynyle renfermant de 2 à 4 atomes de carbone, et que l'on obtient le composé de formule correspondant:

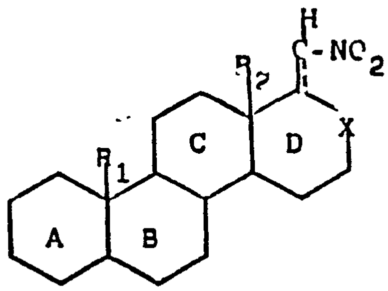

(I)

dans laquelle $R_1$, $R_2$, X, A, B, C et D sont définis comme précédemment.

4. Application selon la revendication 3, caractérisée en ce que le composé cétonique répond à la formule ($II_A$):

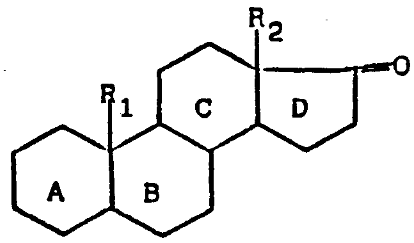

($II_A$)

dans laquelle $R_1$, $R_2$, A, B, C et D sont définis comme précédemment, et que l'on obtient le composé de formule ($I_A$) correspondant:

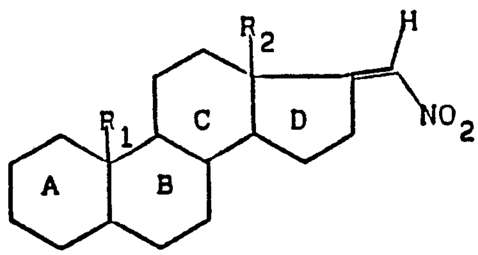

($I_A$)

dans laquelle $R_1$, $R_2$, A, B, C et D sont définis comme précédemment.

5. Application selon la revendication 4, caractérisée en ce que le composé cétonique répond à la formule ($II_A$) dans laquelle $R_2$ représente un radical méthyle et $R_1$ représente un atome d'hydrogène ou un radical méthyle et que l'on obtient le composé de formule ($I_A$) correspondant,

6. Application selon la revendication 5, caractérisée en ce que le composé cétonique répond à la formule ($II'_A$):

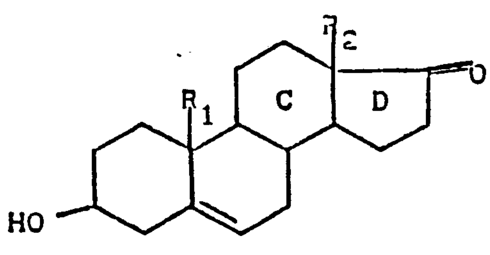

($II'_A$)

dans laquelle $R_1$, $R_2$, C et D sont définis comme précédemment et que l'on obtient le composé de formule ($I'_A$) correspondant:

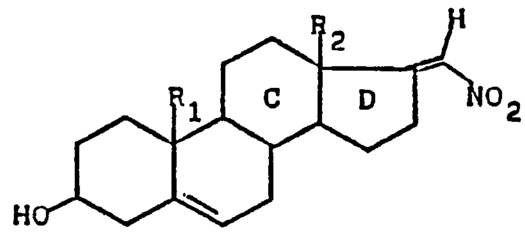

(I'$_A$)

dans laquelle $R_1$, $R_2$, C et D sont définis comme précédemment.

7. Application selon la revendication 6, caractérisée en ce que le composé cétonique répond à la formule (II"$_A$):

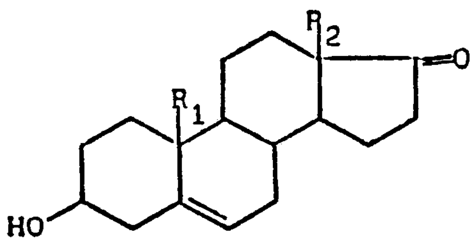

(II"$_A$)

dans laquelle $R_1$ et $R_2$ sont définis comme précédemment et que l'on obtient le composé de formule (I"$_A$) correspondant:

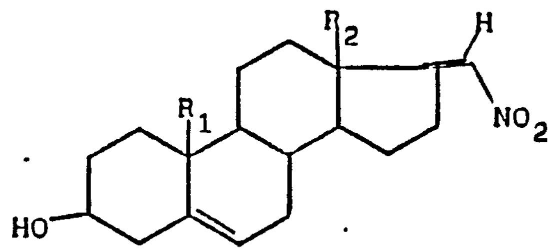

(I"$_A$)

dans laquelle $R_1$ et $R_2$ sont définis comme précédemment.

8. Application selon la revendication 7, caractérisée en ce que le produit de formule II"$_A$ utilisé est la 3β-hydroxy androst-5-en 17-one et le produit de formule I obtenu est le 3β-hydroxy 17-(nitrométhylène) androst-5-ène.

**Revendications** pour l'Etat contractant: AT

1. Procédé de nitrométhylation des composés cétoniques, caractérisé en ce qu'il consiste à utiliser le nitrométhane, en présence d'un catalyseur basique bifonctionnel choisi dans le groupe constitué par l'éthylène diamine, la triméthylène diamine et la tétraméthylène diamine.

2. Procédé selon la revendication 1 , caractérisé en ce que le composé cétonique répond à la formule (II):

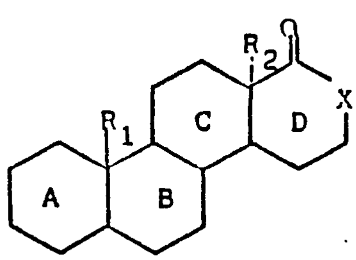

(II)

dans laquelle $R_1$ représente un atome d'hydrogène, un radical alkyle renfermant de 1 à 4 atomes de carbone éventuellement substitué par une fonction oxygénée ou azotée ou par un atome d'halogène, ou $R_1$ représente un radical alkényle ou alkynyle renfermant de 2 à 4 atomes de carbone, $R_2$ représente un radical alkyle renfermant de 1 à 4 atomes de carbone, X représente un atome de carbone ou une liaison carbone-carbone, les noyaux A, B, C et D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle et

alkynyle renfermant de 2 à 4 atomes de carbone, et que l'on obtient le composé de formule (I) correspondant:

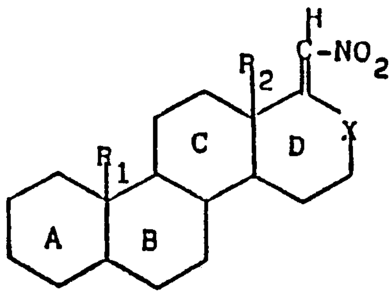

(I)

dans laquelle $R_1$, $R_2$, X, B, C et D sont définis comme précédemment.

3. Procédé selon la revendication 2, caractérisé en ce que le composé cétonique répond à la formule ($II_A$):

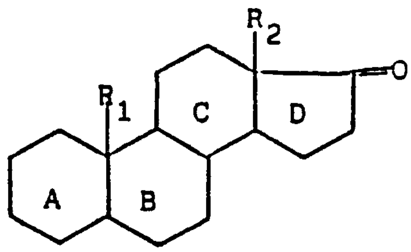

($II_A$)

dans laquelle $R_1$, $R_2$, A, B, C et D sont définis comme précédemment, et que l'on obtient le composé de formule ($I_A$) correspondant:

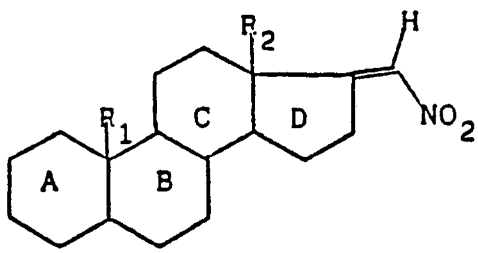

($I_A$)

dans laquelle $R_1$, $R_2$, A, B, C et D sont définis comme précédemment.

4. Procédé selon la revendication 3, caractérisé en ce que le composé cétonique répond à la formule ($II_A$) dans laquelle $R_2$ représente un radical méthyle et $R_1$ représente un atome d'hydrogène ou un radical méthyle et que l'on obtient le composé de formule ($I_A$) correspondant.

5. Procédé selon la revendication 4, caractérisé en ce que le composé cétonique répond à la formule ($II'_A$):

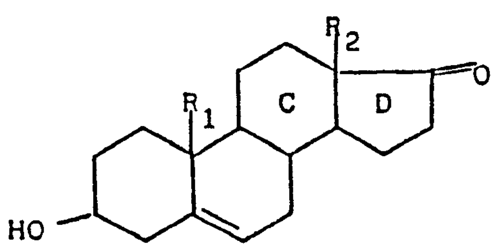

($II'_A$)

dans laquelle $R_1$, $R_2$, C et D sont définis comme précédemment et que l'on obtient le composé de formule ($I'_A$) correspondant:

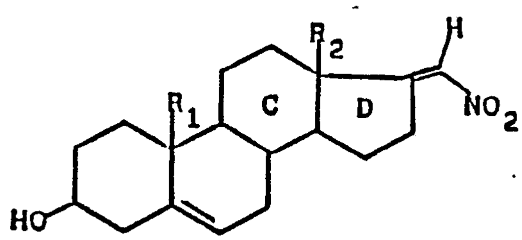

$(I'_A)$

dans laquelle $R_1$, $R_2$, C et D sont définis comme précédemment.

6. Procédé selon la revendication 5, caractérisé en ce que le composé cétonique répond à la formule $(II''_A)$:

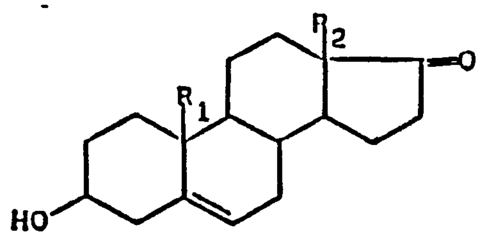

$(II''_A)$'

dans laquelle $R_1$ et $R_2$ sont définis comme précédemment et que l'on obtient le composé de formule $(I''_A)$ correspondant:

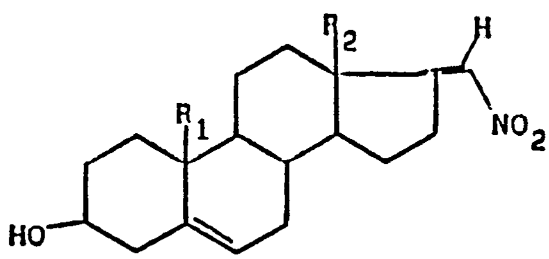

$(I''_A)$

dans laquelle $R_1$ et $R_2$ sont définis comme précédemment.

7. Procédé selon la revendication 6, caractérisé en ce que le produit de formule $II''_A$ utilisé est la 3β-hydroxy androst-5-en 17-one et le produit de formule I obtenu est le 3β-hydroxy 17-(nitrométhylène)androst-5-ène.

**Claims** for the contracting states: CH, DE, FR, GB, IT, LI, NL, SE

1. Reagent for nitromethylation of ketone compounds, characterized in that it contains nitromethane and a bifunctional basic catalyst chosen from ethylene diamine, trimethylene diamine and tetramethylene diamine.

2. Use of the reagent as defined in claim 1 or 2, characterized in that a cyclic or acyclic organic compound, containing at least one ketone group, is submitted to the action of the said reagent, and in this way a nitromethylenated compound is obtained.

3. Use according to claim 2, characterized in that the ketone compound corresponds to the formula (II):

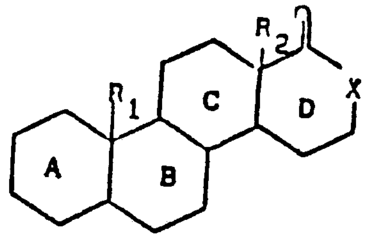

(II)

in which $R_1$ represents a hydrogen atom, an alkyl radical containing from 1 to 4 carbon atoms, optionally substituted by an oxygen-containing or nitrogen-containing function or by a halogen atom, or $R_1$ represents an alkenyl or alkynyl radical containing from 2 to 4 carbon atoms, $R_2$ represents an alkyl radical containing from 1

to 4 carbon atoms, X represents a carbon atom or a carbon-carbon bond, the nuclei A, B, C and D optionally carry one or more double bonds and are optionally substituted by one or more hydroxyl or ketone functions, by one or more halogen atoms, by one or more alkyl or alkyloxy radicals containing from 1 to 4 carbon atoms or by one or more alkenyl and alkynyl radicals containing from 2 to 4 carbon atoms, and in that the corresponding compound of formula (I):

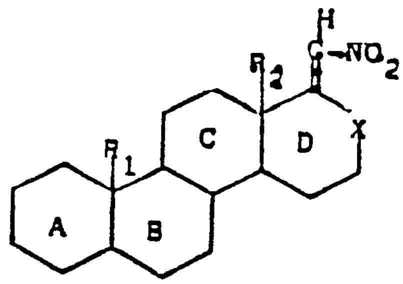

(I)

is obtained, in which $R_1$, $R_2$, X, A, B, C and D are defined as previously.

4. Use according to claim 3, characterized in that the ketone compound corresponds to the formula ($II_A$):

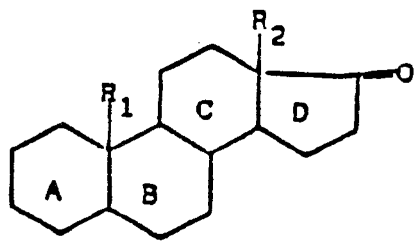

($II_A$)

in which $R_1$, $R_2$, A, B, C and D are defined as previously and in that the corresponding compound of formula ($I_A$):

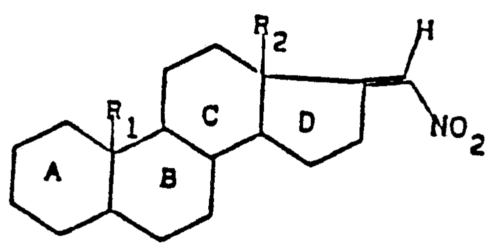

($I_A$)

is obtained, in which $R_1$, $R_2$, A, B, C and D are defined as previously.

5. Use according to claim 4, characterized in that the ketone compound corresponds to the formula ($II_A$) in which $R_2$ represents a methyl radical and $R_1$ represents a hydrogen atom or a methyl radical, and in that the corresponding compound of formula ($I_A$) is obtained.

6. Use according to claim 5, characterized in that the ketone compound corresponds to the formula ($II'_A$):

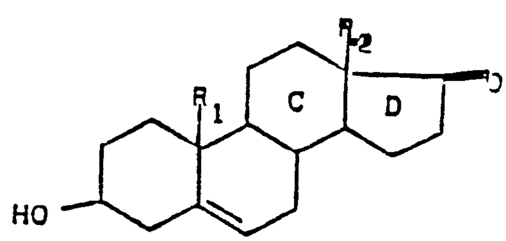

($II'_A$)

in which $R_1$, $R_2$, C and D are defined as previously, and in that the corresponding compound of formula ($I'_A$):

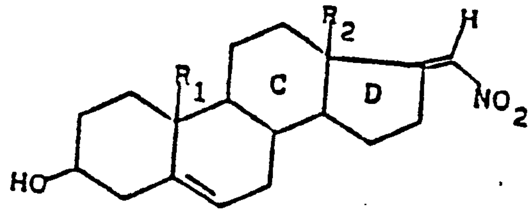

(I'$_A$)

is obtained, in which $R_1$, $R_2$, C and D are defined as previously.

7. Use according to claim 6, characterized in that the ketone compound corresponds to the formula (II''$_A$):

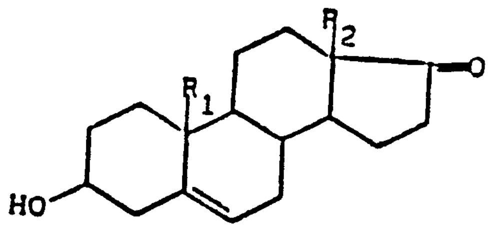

(II''$_A$)

in which $R_1$ and $R_2$ are defined as previously, and in that the corresponding compound of formula (I''$_A$):

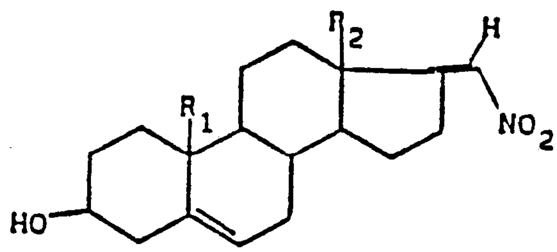

(I''$_A$)

is obtained, in which $R_1$ and $R_2$ are defined as previously.

8. Use according to claim 7, characterized in that the product of formula (II''$_A$) used is 3beta-hydroxy androst-5-en 17-one and the product of formula (I) obtained is 3beta-hydroxy 17-(nitromethylene)-androst-5-ene.

**Claims** for the contracting state: AT

1. Nitromethylation process for ketone compounds, characterized in that it consists of using nitromethane, in the presence of a bifunctional basic catalyst chosen from the group constituted by ethylene diamine, trimethylene diamine and tetramethylene diamine.

2. Process according to claim 1, characterized in that the ketone compound corresponds to the formula (II):

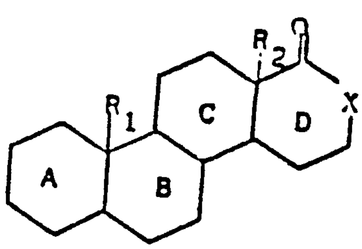

(II)

in which $R_1$ represents a hydrogen atom, an alkyl radical containing from 1 to 4 carbon atoms, optionally substituted by an oxygen-containing or nitrogen-containing function or by a halogen atom, or $R_1$ represents an alkenyl or alkynyl radical containing from 2 to 4 carbon atoms, $R_2$ represents an alkyl radical containing from 1 to 4 carbon atoms, X represents a carbon atom or a carbon-carbon bond, the nuclei A, B, C and D optionally carry one or more double bonds and are optionally substituted by one or more hydroxyl or ketone functions, by one or more halogen atoms, by one or more alkyl or alkyloxy radicals containing from 1 to 4 carbon atoms or by one or more alkenyl and alkynyl radicals containing from 2 to 4 carbon atoms, and in that the corresponding compound of formula (I):

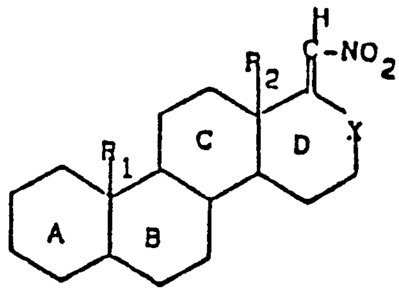

(I)

is obtained, in which $R_1$, $R_2$, X, A, B, C and D are defined as previously.

3. Process according to claim 2, characterized in that the ketone compound corresponds to the formula ($II_A$):

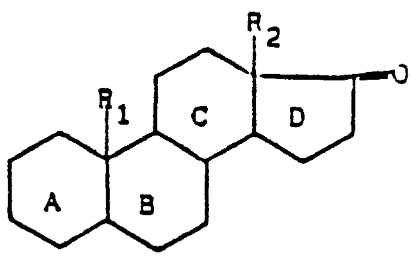

($II_A$)

in which $R_1$, $R_2$, A, B, C and D are defined as previously, and in that the corresponding compound of formula ($I_A$):

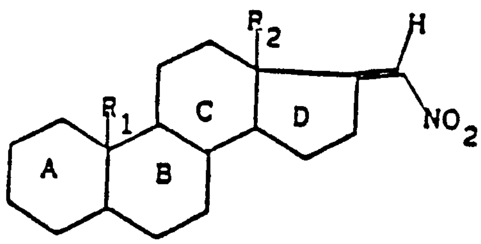

($I_A$)

is obtained, in which $R_1$, $R_2$, A, B, C and D are defined as previously.

4. Process according to claim 3, characterized in that the ketone function corresponds to the formula ($II_A$) in which $R_2$ represents a methyl radical and $R_1$ represents a hydrogen atom or a methyl radical, and in that the corresponding compound of formula ($I_A$) is obtained.

5. Process according to claim 4, characterized in that the ketone compound corresponds to the formula ($II'_A$):

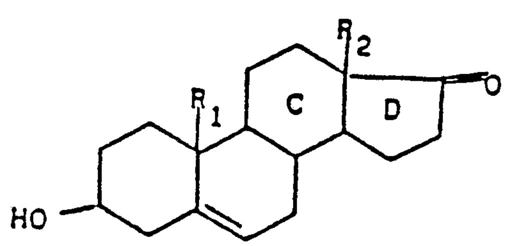

($II'_A$)

in which $R_1$, $R_2$, C and D are defined as previously, and in that the corresponding compound of formula ($I'_A$):

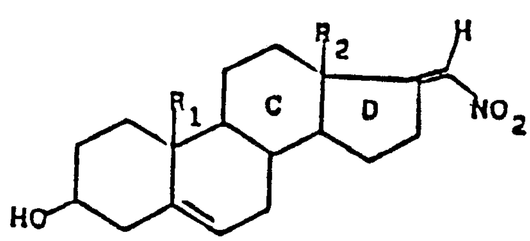

($I'_A$)

is obtained, in which $R_1$, $R_2$, C and D are defined as previously.

6. Process according to claim 5, characterized in that the ketone compound corresponds to the formula ($II''_A$):

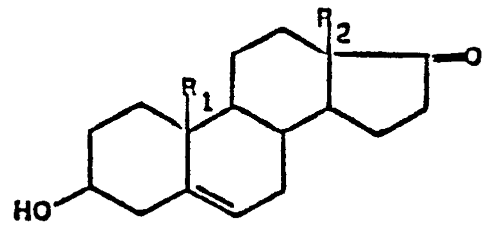

(II"$_A$)

in which $R_1$ and $R_2$ are defined as previously, and in that the corresponding compound of formula (I"A):

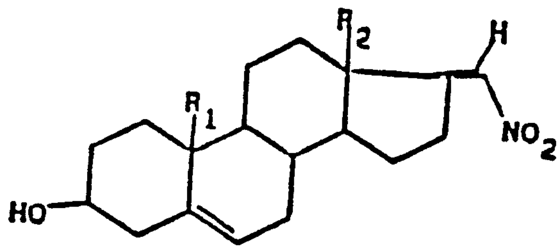

(I"$_A$)

is obtained, in which $R_1$ and $R_2$ are defined as previously.

7. Process according to claim 6, characterized in that the product of formula (II"$_A$) used is 3beta-hydroxy androst-5-en 17-one, and the product of formula (I) obtained is 3beta-hydroxy 17-(nitromethylene)-androst-5-ene.

**Patentansprüche** für die Vertragsstaaten: CH, DE, FR, GB, IT, LI, NL, SE

1. Nitromethylierungs-Reagens für Ketonverbindungen, dadurch gekennzeichnet, daß es Nitromethan und einen bifunktionellen, basischen Katalysator, ausgewählt unter Ethylendiamin, Trimethylendiamin und Tetramethylendiamin, umfaßt.

2. Anwendung des Reagens gemäß Anspruch 1, dadurch gekennzeichnet, daß man eine cyclische oder acyclische, organische Verbindung, die zumindest eine Ketogruppe enthält, der Einwirkung des Reagens unterzieht und so eine nitromethylenierte Verbindung erhält.

3. Anwendung gemäß Anspruch 2, dadurch gekennzeichnet, daß die Ketonverbindung der Formel (II)

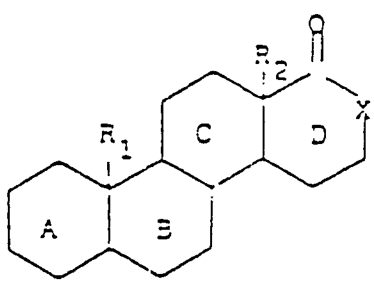

(II)

entspricht, worin $R_1$ für ein Wasserstoffatom, eine Alkylgruppe mit 1 bis 4 Kohlenstoffatomen, die gegebenenfalls substituiert ist durch eine Sauerstoff oder Stickstoff enthaltende Funktion oder durch ein Halogenatom, steht oder $R_1$ für eine Alkenyl- oder Alkinylgruppe mit 2 bis 4 Kohlenstoffatomen steht, $R_2$ eine Alkylgruppe mit 1 bis 4 Kohlenstoffatomen wiedergibt, X ein Kohlenstoffatom oder eine Kohlenstoff-Kohlenstoff-Bindung bedeutet, die Ringe A, B, C und D gegebenenfalls eine oder mehrere Doppelbindungen aufweisen und gegebenenfalls durch eine oder mehrere Hydroxyl- oder Ketofunktionen, durch ein oder mehrere Halogenatome, durch einen oder mehrere Alkyl- oder Alkoxyreste mit 1 bis 4 Kohlenstoffatomen oder durch eine oder mehrere Alkenyl- oder Alkinylgruppen mit 2 bis 4 Kohlenstoffatomen substituiert sind, und daß man die entsprechende Verbindung der Formel (I)

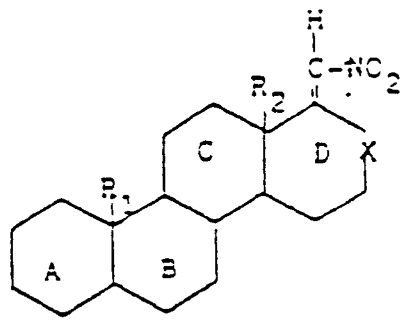

(I)

erhält, worin $R_1$, $R_2$, X, A, B, C und D wie vorstehend definiert sind.

4. Anwendung gemäß Anspruch 3, dadurch gekennzeichnet, daß die Ketonverbindung der Formel ($II_A$)

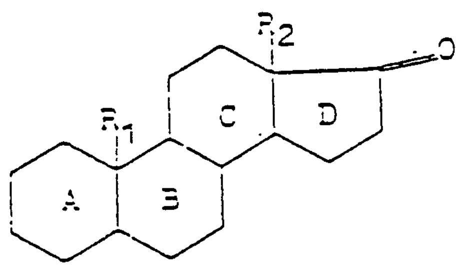

($II_A$)

entspricht, worin $R_1$, $R_2$, A, B, C und D wie vorstehend definiert sind, und daß man die entsprechende Verbindung der Formel ($I_A$)

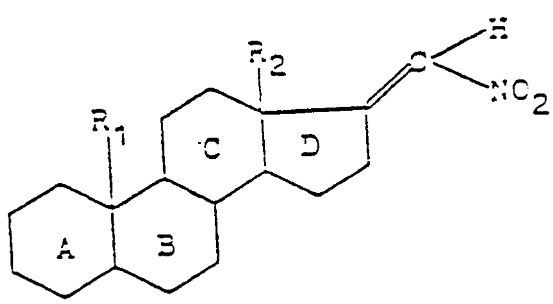

($I_A$)

erhält, worin $R_1$, $R_2$, A, B, C und D wie vorstehend definiert sind.

5. Anwendung gemäß Anspruch 4, dadurch gekennzeichnet, daß die Ketonverbindung der Formel ($II_A$) entspricht, worin $R_2$ für eine Methylgruppe steht und $R_1$ für ein Wasserstoffatom oder eine Methylgruppe steht, und daß man die entsprechende Verbindung der Formel ($I_A$) erhält.

6. Anwendung gemäß Anspruch 5, dadurch gekennzeichnet, daß die Ketonverbindung der Formel ($II'_A$)

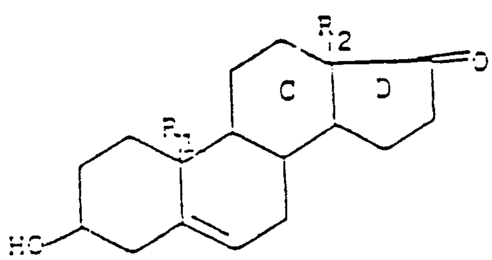

($II'_A$)

entspricht, worin $R_1$, $R_2$, C und D wie vorstehend definiert sind, und daß man die entsprechende Verbindung der Formel ($I'_A$)

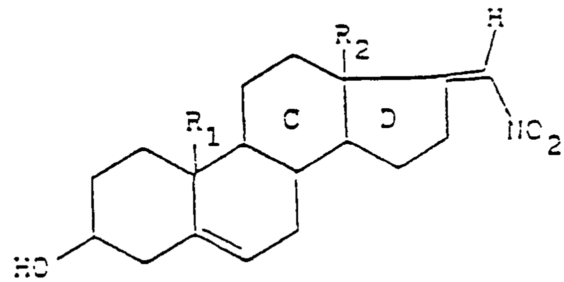

(I'A)

erhält,

worin $R_1$, $R_2$, C und D wie vorstehend definiert sind.

7. Anwendung gemäß Anspruch 6, dadurch gekennzeichnet, daß die Ketonverbindung der Formel ($II''_A$)

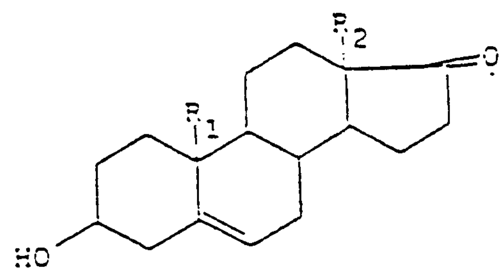

(II"A)

worin $R_1$ und $R_2$ wie vorstehend definiert sind, entspricht und daß man die entsprechende Verbindung der Formel ($I''_A$)

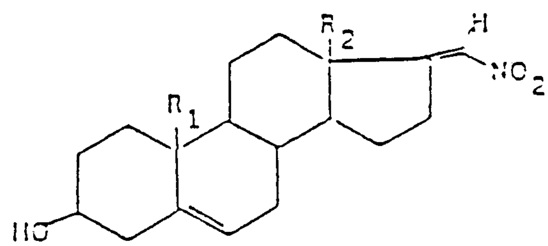

(I"A)

erhält, worin $R_1$ und $R_2$ wie vorstehend definiert sind.

8. Anwendung gemäß Anspruch 7, dadurch gekennzeichnet, daß das verwendete Produkt der Formel $II''_A$ das 3β-Hydroxy-androst-5-en-17-on und das erhaltene Produkt der Formel I das 3β-Hydroxy-17-(nitromethylen)-androst-5-en ist.

**Patentansprüche** für den Vertragsstaat: AT

1. Verfahren zur Nitromethylierung von Ketonverbindungen, dadurch gekennzeichnet, daß man das Nitromethan in Gegenwart eines bifunktionellen, basischen Katalysators, ausgewählt unter Ethylendiamin, Trimethylendiamin und Tetramethylendiamin, einsetzt.

2. Verfahren gemäß Anspruch 1, dadurch gekennzeichnet, daß die Ketonverbindung der Formel (II)

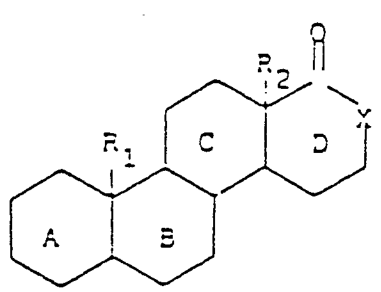

(II)

entspricht, worin $R_1$ für ein Wasserstoffatom, eine Alkylgruppe mit 1 bis 4 Kohlenstoffatomen, die gegebenenfalls substituiert ist durch eine Sauerstoff oder Stickstoff enthaltende Funktion oder durch ein Halogenatom, steht oder $R_1$ für eine Alkenyl- oder Alkinylgruppe mit 2 bis 4 Kohlenstoffatomen steht, $R_2$ eine

Alkylgruppe mit 1 bis 4 Kohlenstoffatomen wiedergibt, X ein Kohlenstoffatom oder eine Kohlenstoff-Kohlenstoff-Bindung bedeutet, die Ringe A, B, C und D gegebenenfalls eine oder mehrere Doppelbindungen aufweisen und gegebenenfalls durch eine oder mehrere Hydroxyl- oder Ketofunktionen, durch ein oder mehrere Halogenatome, durch einen oder mehrere Alkyl- oder Alkoxyreste mit 1 bis 4 Kohlenstoffatomen oder durch eine oder mehrere Alkenyl- oder Alkinylgruppen mit 2 bis 4 Kohlenstoffatomen substituiert sind, und daß man die entsprechende Verbindung der Formel (I)

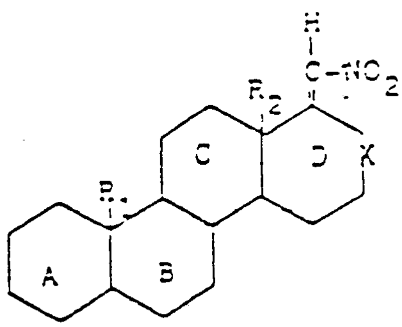

(I)

erhält, worin $R_1$, $R_2$, X, B, C und D wie vorstehend definiert sind.

3. Verfahren gemäß Anspruch 2, dadurch gekennzeichnet, daß die Ketonverbindung der Formel ($II_A$)

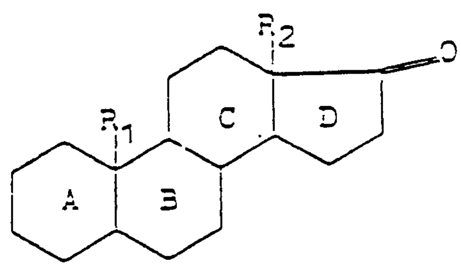

($II_A$)

entspricht, worin $R_1$, $R_2$, A, B, C und D wie vorstehend definiert sind, und daß man die entsprechende Verbindung der Formel ($I_A$)

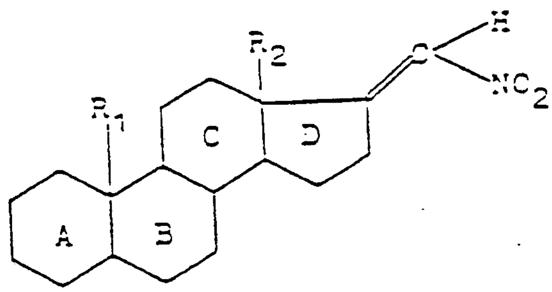

($I_A$)

erhält, worin $R_1$, $R_2$, A, B, C und D wie vorstehend definiert sind.

4. Verfahren gemäß Anspruch 3, dadurch gekennzeichnet, daß die Ketonverbindung der Formel ($II_A$) entspricht, worin $R_2$ für eine Methylgruppe steht und $R_1$ für ein Wasserstoffatom oder eine Methylgruppe steht, und daß man die entsprechende Verbindung der Formel ($I_A$) erhält.

5. Verfahren gemäß Anspruch 4, dadurch gekennzeichnet, daß die Ketonverbindung der Formel ($II'_A$)

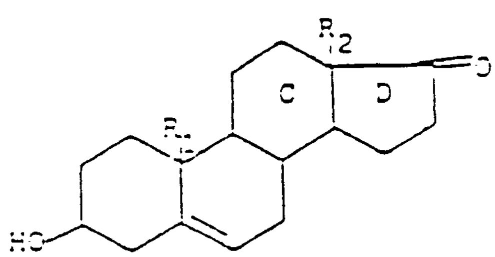

($II'_A$)

entspricht worin $R_1$ $R_2$, C und D wie vorstehend definiert sind, und daß man die entsprechende Verbindung der Formel ($I'_A$)

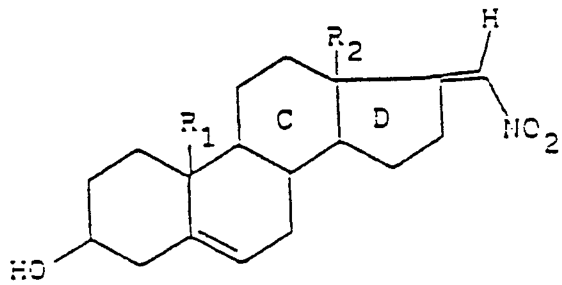

($I'_A$)

erhält, worin $R_1$, $R_2$, C und D wie vorstehend definiert sind.

6. Verfahren gemäß Anspruch 3, dadurch gekennzeichnet, daß die Ketonverbindung der Formel ($II''_A$)

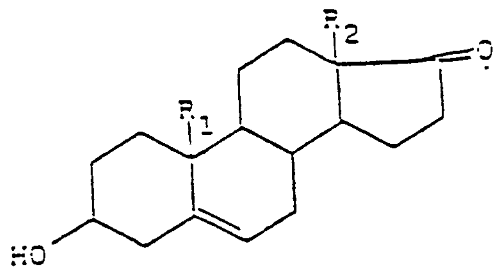

($II''_A$)

worin $R_1$ und $R_2$ wie vorstehend definiert sind, entspricht und daß man die entsprechende Verbindung der Formel ($I''_A$)

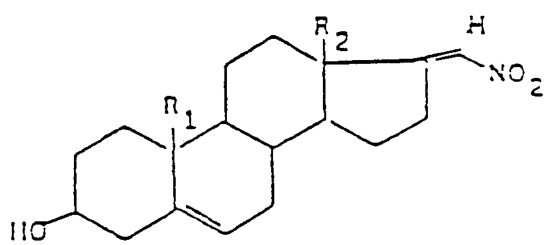

($I''_A$)

erhält, worin $R_1$ und $R_2$ wie vorstehend definiert sind.

7. Verfahren gemäß Anspruch 6, dadurch gekennzeichnet, daß das verwendete Produkt der Formel $II''_A$ das 3β-Hydroxy-androst-5-en-17-on und das erhaltene Produkt der Formel I das 3β-Hydroxy-17-(nitromethylen)-androst-5-en ist.